# EUROPÄISCHE PATENTSCHRIFT

(11) **EP 1 380 583 B1**
(45) Veröffentlichungstag und Bekanntmachung des Hinweises auf die Patenterteilung: **01.07.2009**
(21) Anmeldenummer: 03103347.5
(22) Anmeldetag: 14.11.1996
(51) Int. Cl.: A61K 8/00

(54) **Bis-Resorcinyl-Triazine und deren Verwendung als Sonnenschutzmittel**
Bis-resorcinol-triazines and their use as sunscreen agents
Bis-résorcinol-triazines et leur utilisation en tant qu'agent antisolaire

(30) Priorität: 23.11.1995 DE 19543730
(43) Veröffentlichungstag der Anmeldung: 14.01.2004
(62) Teilanmeldung aus: 96810796.1
(73) Patentinhaber: Ciba Holding Inc., 4057 Basel (CH)
(72) Erfinder: Hüglin, Dietmar, 79591, Eimeldingen (DE); Borsos, Elek, 4127, Birsfelden (CH); Luther, Helmut, 79639, Grenzach-Wyhlen (DE); Herzog, Bernd, 79639, Grenzach-Wyhlen (DE); Bachmann, Frank, 79104, Freiburg (DE)

(56) Entgegenhaltungen:
- EP-A- 0 165 608
- DE-A- 4 340 725
- GB-A- 2 286 774

## Beschreibung

Die vorliegende Erfindung betrifft neue Bis-Resorcinyl-Triazine, Verfahren zur Herstellung dieser Verbindungen sowie die Verwendung von ausgewählten Bis-Resorcinyl-Triazinen für kosmetische Mittel.

Die neuen Bis-Resorcinyl-Triazine entsprechen der Formel 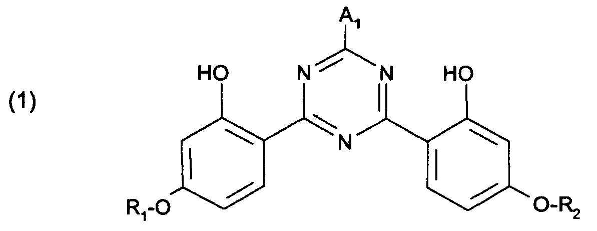 worin
- R₁ und R₂,: unabhängig voneinander, C₃-C₁₈-Alkyl;
- A₁: einen Rest der Formel (1d) 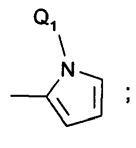 und
- Q₁: C₁-C₁₈-Alkyl
bedeuten.

C₁-C₅-Alkyl, C₁-C₈-Alkyl, C₁-C₁₀-Alkyl, bzw C₃-C₁₈-Alkyl sind geradkettige oder verzweigte Alkylreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Decyl, Undecyl, Dodecyl, Tetradecyl, Pentadecyl, Hexadecyl, Heptadecyl oder Octadecyl.

Als Beispiel für Verbindungen der Formel (1) sei genannt:
2,4-Bis-{[4-(2-ethyl-hexyloxy)-2-hydroxy]-phenyl}-6-(1-methylpyrrol-2-yl)-1,3,5-triazin.

Verbindungen der Formel (1), worin A₁ einen Rest der Formel (1d) und R₁ und R₂ die gleiche Bedeutung haben, lassen sich z.B. in einer dreistufigen Reaktion, ausgehend von Cyanurchlorid, herstellen. Dabei setzt man das entsprechende N-Alkyl-Pyrrol mit Cyanurchlorid in einer Friedel-Crafts-Reaktion selektiv zur Dichlortriazinverbindung der Formel 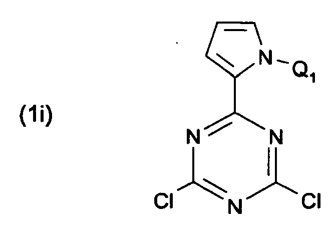 um. Q₁ hat dabei die in Formel (1) angegebene Bedeutung.

Anschliessend werden die beiden Resorcingruppen in allgemein bekannter Weise durch Friedel-Crafts-Acylierung von Resorcin in Gegenwart einer Lewis-Säure, insbesondere Aluminiumchlorid, eingeführt. Diese Reaktionen sind z.B. in der EP-A-165,608 beschrieben. Die Veretherung der freien, p-ständigen Hydroxylgruppen erfolgt durch Alkylierung bzw. säurekatalysierte Addition von Glycidylethern. Detaillierte Angaben dazu kön nen den Synthesebeispielen entnommen werden.

Die erfindungsgemässen Verbindungen der Formel (1) sowie weitere, aus dem Stand der Technik bekannte ausgewählte Bis-Resorcinyl-Triazinverbindungen eignen sich insbesondere als UV-Filter, d.h. zum Schützen von ultraviolett empfindlichen organischen Materialien, insbesondere der Haut und Haare von Menschen und Tieren vor der schädigenden Einwirkung von UV-Strahlung. Diese Verbindungen eignen sich daher als Lichtschutzmittel in kosmetischen, pharmazeutischen und veterinärmedizinischen Präparaten. Diese Verbindungen können sowohl gelöst als auch im mikronisierten Zustand verwendet werden.

Einen weiteren Erfindungsgegenstand bildet daher ein kosmetisches Präparat, enthaltend mindestens eine Verbindung der Formel 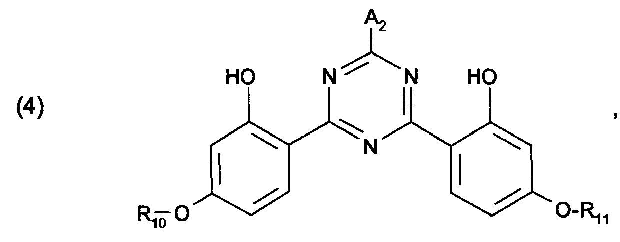 worin
- R₁₀ und R₁₁,: unabhängig voneinander, Wasserstoff; C₃-C₁₈-Alkyl;
- A₂: einen Rest der Formel (4d) 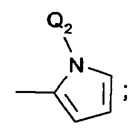 und
- Q₂: C₁-C₁₈-Alkyl;
bedeuten
sowie kosmetisch verträgliche Träger- oder Hilfsstoffe.

Für die kosmetische Verwendung haben die erfindungsgemässen Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die unlöslichen erfindungsgemässen UV-Absorber können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe wie z.B. eines alkylierten Vinylpyrrolidon-Polymers, eines Vinylpyrrolidon-Vinylacetat-Copolymers, eines Acylglutamates oder insbesondere eines Phospholipids durchgeführt.

Die kosmetischen Zusammensetzungen können neben den erfindungsgemässen UV-Absorbern auch noch einen oder mehrere weitere UV-Schutzstoffe, wie z.B. Triazine, Oxanilide, Triazole oder Vinylgruppen enthaltende Amide oder Zimtsäureamide enthalten.

Solche Schutzstoffe sind z.B. in der GB-A-2,286,774 beschrieben oder auch aus Cosmetics & Toiletries (107), 50ff (1992) bekannt.

Die erfindungsgemässen kosmetische Zusammensetzungen enthalten 0,1 bis 15, vorzugsweise 0,5 bis 10 Gew.-%, bezogen auf das Gesamtgewicht der Zusammensetzung, eines UV-Absorbers oder eines Gemisches aus UV-Absorbern und einen kosmetisch verträglichen Hilfsstoff.

Die Herstellung der kosmetischen Zusammensetzungen kann durch physikalisches Mischen des oder der UV-Absorber mit dem Hilfsstoff durch gewöhnliche Methoden, wie z.B. durch einfaches Zusammenrühren der Einzelkomponenten erfolgen.

Die erfindungsgemässen kosmetischen Zusammensetzungen können als Wasser-in-Öloder Öl-in-Wasser-Emulsion, als Öl-in-Alkohol-Lotion, als vesikulare Dispersion eines ionischen oder nichtionischen amphiphilen Lipids, als Gel, fester Stift oder als AerosolFormulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Für die erfindungsgemässen kosmetischen Zusammensetzungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Die kosmetischen Zusammensetzungen können auch weitere Komponenten wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft- und Farbstoffe enthalten.

Die erfindungsgemässen kosmetischen Formulierungen zeichnen sich durch exzellenten Schutz der menschlichen Haut gegen den schädigenden Einfluss von Sonnenlicht aus.

In den folgenden Beispielen beziehen sich die Prozentsätze auf das Gewicht. Die Mengen beziehen sich bei den eingesetzten Bis-Resorcinyl-Triazinverbindungen auf die Reinsubstanz.

### Herstellungsbeispiele der neuen Verbindungen:

### Beispiel 1: 2,4-Bis-{[4-(2-ethyl-hexyloxy)-2-hydroxy]-phenyl}-6-(1-methylpyrrol-2-yl)-1,3,5-triazin

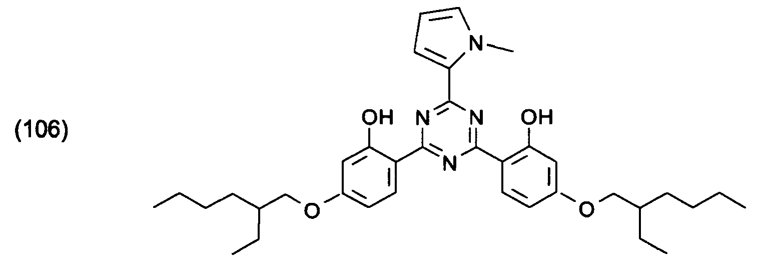

a) 2,4-Dichlor-6-(1-methylpyrrol-2-yl)-1,3,5-triazin: In einem 500ml Sulfierkolben mit Rührer, Tropftrichter, Kühler und Innenthermometer werden 46,1g (0,25 Mol) Cyanurchlorid in 200ml Toluol vorgelegt und langsam mit 21,1g (0,26 Mol) 1-Methyl-pyrrol versetzt. Man erwärmt auf Rückflusstemperatur (110-114°C) und rührt den Ansatz 6 Stunden, bis die Entwicklung von HCl beendet ist. Nach dem Erkalten wird zunächst mit verd. Sodalösung, danach mit Wasser ausgeschüttelt. Man trocknet die organische Phase über Natriumsulfat, dampft zur Trockene ein und verrührt den Rückstand in 750ml n-Hexan. Die Kristalle werden abgesaugt und bei 60°C im Vakuum getrocknet. Ausbeute: 23,0g (40,2% d.Th.).

### Eigenschaften:

### leicht braune Kristalle; Smp.: 155-156°C.

b) Friedel-Crafts-Acylierung: In einem 500ml Sulfierkolben mit Rührer, Tropftrichter, Kühler und Innenthermometer werden 22,9g (0,1 Mol) 2,4-Dichlor-6-(1-methylpyrrol-2-yl)-1,3,5-triazin und 24,2g (0,22 Mol) Resorcin in 200ml Xylol (Isomerengemisch) vorgelegt. Bei 45-50°C trägt man langsam 29,3g (0,22 Mol) Aluminiumchlorid ein und rührt bei 80-83°C, bis die HCl-Entwicklung beendet ist (ca. 2 Stunden). Man lässt die warme Reaktionslösung unter Rühren in verdünnter Salzsäure einlaufen, saugt ab und wäscht mit Aceton/Wasser neutral. Die Trocknung erfolgt bei 100°C im Vakuum. Man erhält 30,5g (81,0% d.Th.) 2,4-Bis-(2,4-dihydroxy-phenyl)-6-(1-methylpyrrol-2-yl)-1,3,5-triazin als gelbes Pulver (Smp. > 300°C). Die Struktur steht im Einklang mit dem ¹H-NMR-Spektrum.
c) Alkylierung: In einem 250ml Sulfierkolben mit Rührer, Tropftrichter, Kühler und Innenthermometer werden 7,53g (0,02 Mol) 2,4-Bis-(2,4-dihydroxy-phenyl)-6-(1-methylpyrrol-2-yl)-1,3,5-triazin zusammen mit 80ml Methylcellosolve (Merck®) und 5,6g (0,04 Mol) 30%-ige Natronlauge bei Raumtemperatur vorgelegt. Man rührt 30 Minuten lang bei 80°C und tropft bei derselben Temperatur 9,5g (0,048 Mol) 3-Brommethyl-heptan, gelöst in 25ml Methylcellosolve, langsam zu. Die Alkylierung lässt sich dünnschichtchromatographisch verfolgen. Nach 6 Stunden Reaktion bei 110-112°C kann das Edukt nicht mehr nachgewiesen werden. Man dampft zur Trockene ein, nimmt in 100ml Toluol / Aceton (7 vol./3 vol.) auf und filtriert vom Ungelösten ab. Zur Reinigung wird über Kieselgel (Säule: Ø=5cm, I=60cm) chromatographiert. Man erhält die Verbindung der Formel (106) als zähes, leicht gelbes Harz, welches nach einiger Zeit auskristallisiert. Ausbeute: 5,8g (48,3% d.Th.). Die Kristallisation kann durch Zugabe von Impfkristallen beschleunigt werden.

Eigenschaften: schwach gelbe Kristalle; Smp.: 95-96°C
UV-Spektrum: λₘₐₓ = 349nm, λₘₐₓ = 71 000 M⁻¹cm⁻¹ (Ethanol)
¹H-NMR-Spektrum: δ [ppm, CDCl₃] = 0,8-1,0 (m, 12H, -CH₃), 1,2-1,8 (m, 18H, -CH₂- und - CH-), 3,8-3,9 (d, 4H, -O-CH₂-), 4,15 (s, 3H, N-CH₃), 6,4-8,6 (9H, Aromaten), 13,5 (s, 2H, -OH)

### Applikationsbeispiele

### Anwendungsbeispiele für kosmetischen Lichtschutz

Die Lichtschutzfaktoren wurden bestimmt gemäss der Methode von Diffey und Robson, J. Soc. Cosmet. Chem. 40, 127 - 133 (1989) mit einem SPF-Analysator (Optometrix, SPF 290).

Zur Bestimmung der Photostabilitäten werden die Filtersubstanzen in Ethanol gelöst (c = 1·10⁻⁵ - 5·10⁻⁵ M) und in einer Quartz-Küvette unter Rühren mit einer Metallhalogenidlampe (Macam) bestrahlt (I_{UVB}= 0,4 - 8,0 mW/cm²). Zur Umrechnung auf das Sonnenspektrum (CIE D65-Normtageslicht, normiert auf I_{UVB}= 0,127 mW/cm²) wird das Integral über die Produkte der wellenlängenaufgelösten Lampenintensität mit den entsprechenden Absorptionswerten des jeweiligen UV-Absorbers zwischen 290 und 400 nm berechnet und durch das Integral über die Produkte der D65-Lichtintensitäten mit den entsprechenden Absorptionswerten des jeweiligen UV-Absorbers im Bereich zwischen 290 und 400 nm dividiert. Mit diesem Faktor wird die Halbwertszeit für den Abbau unter Bestrahlung mit der Metallhalogenidlampe multipliziert, um die entsprechende Halbwertszeit unter Sonneneinstrahlung zu erhalten. Die Halbwertszeit für den Photoabbau unter Lampeneinstrahlung wird über UVspektroskopische Messung der Extinktion bei der Wellenlänge der Maximalabsorption und anschliessenden Exponentialfit bestimmt. Mit dem beschriebenen Verfahren erhält man also die Halbwertszeiten für den Photoabbau im D65-Licht.

### Beispiel 2: o/w-Emulsion mit der Verbindung der Formel (106)

| (A): | |
|---|---|
| Triazin-UV-Absorber | 3 g |
| Sesamöl | 10 g |
| Glycerylstearat | 4 g |
| Stearinsäure | 1 g |
| Cetylalkohol | 0,5 g |
| Polysorbat 20 | 0,2 g |

| (B): | |
|---|---|
| Propylenglykol | 4 g |
| Propylparaben | 0,05 g |
| Methylparaben | 0,15 |
| Triethanolamin | 0,1 g |
| Carbomer 934 | 0,1 g |
| Wasser | ad 100 ml |

### Herstellung der Emulsion

### Phase (A):

Zunächst wird der UV-Absorber in Sesamöl gelöst. Die anderen Komponenten von (A) werden dazugegeben und zusammengeschmolzen.

### Phase (B):

Propylparaben und Methylparaben werden im Propylenglykol gelöst. Danach werden 60ml Wasser zugegeben, auf 70°C erhitzt und Carbomer 934 darin emulgiert.

### Emulsion:

(A) wird langsam unter starkem mechanischem Energieeintrag zu B gegeben. Das Volumen wird durch Zugabe von Wasser auf 100 ml eingestellt.

Die ermittelten Sonnenschutzfaktoren und Photostabilitäten sind aus Tabelle 1 zu entnehmen.

| Tabelle 1: | Konzentration | Sonnenschutzfaktor*) | Photostabilität*) [h] |
|---|---|---|---|
| Verbindung der Formel (106) | 3% | 6,2 | 360 |

| | | | |
|---|---|---|---|
| *) nach Diffey und Robson **) als Halbwertszeit des Photoabbaus im D65-Licht in ethanolischer Lösung | | | |

Der Sonnenschutzfaktor kann mit der UV-Absorber-Konzentration variiert werden.

Die Ergebnisse zeigen, dass die Wirksubstanzen eine hohe Photostabilität aufweisen und schon mit niedriger Konzentration ein guter Sonnenschutzfaktor erzielt werden kann.

## Patentansprüche

1. Bis-Resorcinyl-Triazine der Formel 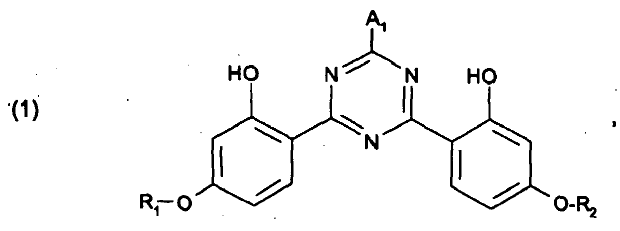 worin R₁ und R₂, unabhängig voneinander, C₃-C₁₈-Alkyl;
A₁ einen Rest der Formel (1d) 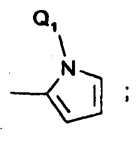 und Q₁ C₁-C₁₈-Alkyl;
bedeuten.

2. 2,4-Bis-{[4-(2-ethyl-hexyloxy)-2-hydroxy]-phenyl}-6-(1-methylpyrrol-2-yl)-1,3,5-triazin.

3. Verfahren zur Herstellung der Verbindungen der Formel (1) gemäss Anspruch 1, wenn A₁ einen Rest der Formel (1d) und R₁ und R₂ die gleiche Bedeutung haben durch selektive Umsetzung der entsprechenden N-Alkyl-Pymol mit Cyanurchlorid in einer Friedel-Crafts-Reaktion zur Dichlortriazinverbindung der Formel 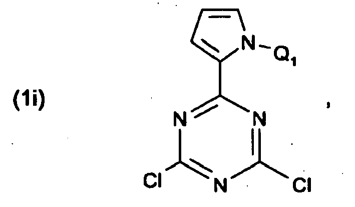 Einführung der Resorcingruppen durch Friedel-Crafts-Acylierung von Resorcin in Gegenwart einer Lewis-Säure, insbesondere Aluminiumchlorid, und Veretherung der freien, p-ständigen Hydroxylgruppen, je nach Bedeutung der Reste R₁ und R₂, durch Alkylierung bzw. säurekatalysierte Addition von Glycidylethern, wobei Q₁ die in Anspruch 1 angegebene Bedeutung hat.

4. Kosmetisches Präparat, enthaltend mindestens eine oder mehrere Verbindungen der Formel (1) nach Anspruch 1 mit kosmetisch verträglichen Träger- oder Hilfsstoffen.

5. Präparat nach Anspruch 4, **dadurch gekennzeichnet, dass** es weitere UV-Schutzstoffe enthält.

6. Präparat nach Anspruch 4 oder 5, **dadurch gekennzeichnet, dass** es als weitere UV-Schutzstoffe Triazine, Oxanilide, Triazole, Vinylgruppen enthaltende Amide oder Zimtsäureamide enthält.

## Claims

1. A bis(resorcinyl)triazine of the formula 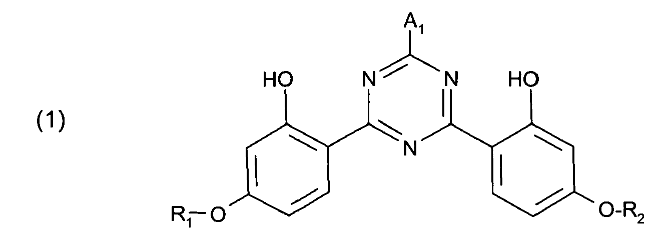 in which
R₁ and R₂, independently of one another, are C₃-C₁₈alkyl;
A₁ is a radical of the formula (1d) 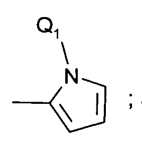 and
Q₁ is C₁-C₁₈alkyl.

2. 2,4-bis{[4-(2-ethylhexyloxy)-2-hydroxy]phenyl}-6-(1-methylpyrrol-2-yl)-1,3,5-triazine.

3. A process for the preparation of the bis(resorcinyl)triazines of the formula (1) according to claim 1, in which A₁ is a radical of the formula (1d) and R₁ and R₂ have the same meaning, by selective reaction of the corresponding N-alkylpyrrole with cyanuric chloride in a Friedel-Crafts reaction to give the dichlorotriazine compound of the formula 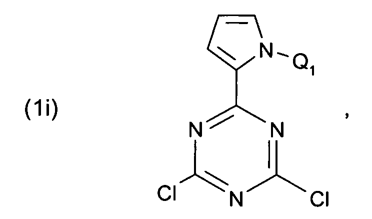 introduction of the resorcinol groups by Friedel-Crafts acylation of resorcinol in the presence of a Lewis acid, in particular aluminium chloride, and etherification of the free p-hydroxyl groups, depending on the meaning of the radicals R₁ and R₂, by alkylation or acid-catalysed addition of glycidyl ethers, wherein
Q₁ is dfined as in claim 1.

4. A cosmetic preparation, comprising at least one or more compounds of the formula (1) according to claim 1 with cosmetically tolerable carriers or auxiliaries.

5. A preparation according to claim 4, which contains further UV-protective substances.

6. A preparation according to claim 4 or 5, which, as further UV-protective substances, contains triazines, oxanilides, triazoles, amides containing vinyl groups or cinnamides.

## Revendications

1. Bis-resorcinyl-triazine présentant la formule: 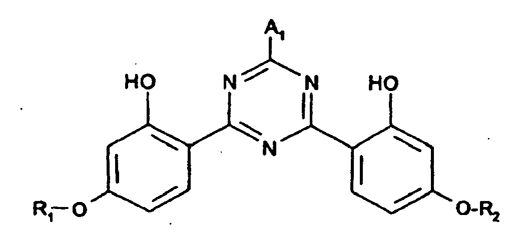 dans laquelle
R₁ et R₂, indépendamment l'un de l'autre, sont des radicaux (C₃-C₁₈)-alkyl ;
A₁ est un résidu de 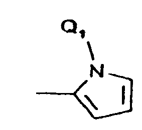 formule (1d)
et
Q₁ est un radical (C₁-C₁₈)-Alkyl.

2. 2,4-Bis-{[4-(2-éthyl-hexyloxy)-2-hydroxy]-phényl}-6-(1-méthylpyrrol-2-yl)-1,3,5-triazine.

3. Procédé de production de composés de formule (1) selon la revendication 1, lorsque A, est un résidu de Formule (1d) et R₁ et R₂ ont la même signification, par transformation sélective du N-alkyl-pyrrol correspondant avec du chlorure de cyanure par réaction de Friedel-Crafts pour l'obtention d'un composé dichlortriazine de formule 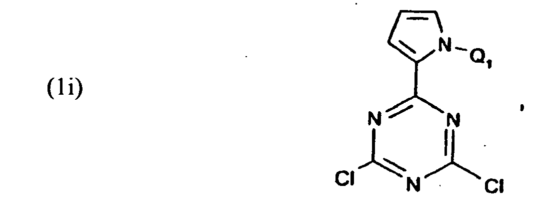 l'introduction par acylation Friedel-Crafts de résorcine de groupes résorciniques en présence d'un acide de Lewis, notamment du chlorure d'aluminium, et une éthérification du groupe hydroxyle libre en position para, selon la signification des résidus R₁ et R₂, par alkylation ou addition catalysée par des éthers glycidyliques, Q₁ ayant la signification indiquée dans la revendication 1 précitée.

4. Préparation cosmétique, contenant au moins un ou plusieurs des composés présentant la formule (1) selon la revendication 1 combinée à un support ou adjuvant acceptable du point de vue cosmétique.

5. Préparation selon la revendication 4, **caractérisée en ce qu'**elle contient en outre un agent de protection contre les UV.

6. Préparation selon la revendication 4 ou 5, **caractérisée en ce que**, en tant qu'agent de protection contre les UV, elle contient des triazine, oxanilide, triazole, amides d'acide cinnamique ou des amides comportant des groupes vinyliques.
